# EUROPÄISCHE PATENTSCHRIFT

(11) **EP 2 797 588 B1**
(45) Veröffentlichungstag und Bekanntmachung des Hinweises auf die Patenterteilung: **27.04.2016**
(21) Anmeldenummer: 12810316.5
(22) Anmeldetag: 28.12.2012
(51) Int. Cl.: A61K 9/70

(54) **TRANSDERMALES THERAPEUTISCHES SYSTEM MIT GERINGER NEIGUNG ZUR SPONTANKRISTALLISATION**
TRANSDERMAL THERAPEUTIC SYSTEM WITH A LOW TENDENCY TO SPONTANEOUSLY CRYSTALLIZE
SYSTÈME THÉRAPEUTIQUE TRANSDERMIQUE PRÉSENTANT UNE TENDANCE RÉDUITE À LA CRISTALLISATION SPONTANÉE

(30) Priorität: 30.12.2011 DE 102011090178
(43) Veröffentlichungstag der Anmeldung: 05.11.2014
(73) Patentinhaber: UCB Pharma GmbH, 40789 Monheim (DE); LTS LOHMANN Therapie-Systeme AG, 56626 Andernach (DE)
(72) Erfinder: DZEKAN, Horst, 56584 Meinborn (DE); HOFFMANN, Hans-Rainer, 56566 Neuwied (DE); HORSTMANN, Michael, 56564 Neuwied (DE); MÜLLER, Walter, 56626 Andernach (DE); WIEDERSBERG, Sandra, 06268 Steigra (DE)
(74) Vertreter: Hoffmann Eitle
(86) Internationale Anmeldenummer: PCT/EP2012/077046
(87) Internationale Veröffentlichungsnummer: WO 2013/098390

(56) Entgegenhaltungen:
- EP-A1- 0 391 172
- WO-A1-2007/140909

## Beschreibung

Die vorliegende Erfindung betrifft transdermale therapeutische Systeme zur Abgabe von Arzneistoffen, wobei die transdermalen therapeutischen Systeme eine geringe Neigung zur spontanen Kristallisation des in ihnen enthaltenen Arzneistoffs aufweisen.

Transdermale therapeutische Systeme (TTS) sind seit einigen Jahren bekannt und mittlerweile im Arzneimittelmarkt eingeführt. Es gibt transdermale therapeutische Systeme, die den Arzneistoff in gelöster Form in einer einschichtigen oder mehrschichtigen Polymermatrix enthalten. Daneben sind auch Systeme erhältlich, die einen geschichteten Aufbau mit einem Reservoir, einer die Arzneistoffabgabe steuernde Membran und einer Klebschicht aufweisen. Dazu kommen andere Systeme, die noch weiter spezialisierte Funktionsmerkmale aufweisen.

Fast alle heute kommerziell erhältlichen transdermalen therapeutischen Systeme enthalten den Arzneistoff in gelöster Form. Bei den allermeisten dieser Systeme liegt der Arzneistoffgehalt in jeder Schicht unterhalb der Sättigungslöslichkeit bei Raumtemperatur für den Arzneistoff in der entsprechenden Schicht, damit der Arzneistoff nicht kristallisiert. Würde der Arzneistoffgehalt in einer Schicht über der Sättigungslöslichkeit für den Arzneistoff in dieser Schicht liegen, bestünde die Gefahr der Kristallisation des Arzneistoffs. Mit dieser Instabilität wäre eine geringere thermodynamische Aktivität des Arzneistoffs verbunden, die zu einer verringerten Arzneistoffabgabe aus dem TTS führt.

Eines der Probleme bei der Entwicklung von transdermalen therapeutischen Systemen besteht somit darin, dass sie eine möglichst hohe Arzneistoffkonzentration aufweisen sollten, um eine hohe Arzneistoffabgabe bei vergleichsweise kleiner Abgabefläche erreichen zu können, andererseits aber auch die Stabilität eines TTS über eine lange, gegebenenfalls mehrere Jahre dauernde Lagerzeit gesichert sein muss. Insbesondere gilt es, das Risiko einer Kristallisation des Arzneistoffs in dem TTS aufgrund seiner Übersättigung zu vermeiden. Das Dilemma bei der Entwicklung transdermaler therapeutischer Systeme besteht somit darin, möglichst hohe Arzneistoffkonzentrationen einzusetzen, ohne das Risiko einer Kristallisation des Arzneistoffs im TTS eingehen zu müssen.

Es wurden zahlreiche Lösungsvorschläge unterbreitet, um mit Arzneistoff übersättigte TTS zu stabilisieren und ein Kristallisieren des in ihnen enthaltenen Arzneistoffs zu vermeiden. Insbesondere scheinen verschiedene Hilfsstoffe dafür geeignet zu sein, die dem TTS, insbesondere der arzneistoffhaltigen Matrix, bei seiner Herstellung zugesetzt werden.

In der EP 0 391 172 A1 werden transdermale therapeutische Systeme mit einem Schichtaufbau beschrieben, der aus einer wirkstoffundurchlässigen Rückschicht, einer Matrix mit darin verteilten Inseln, die den Arzneistoff enthalten, und einer den Zutritt von Hautfeuchtigkeit kontrollierenden Schicht besteht. Die Matrix basiert auf einem wasserdampfdurchlässigen, im Wesentlichen wasserunlöslichen und weitgehend wirkstofffreien Grundmaterial, beispielsweise Silikonpolymeren. Die in dem Grundmaterial der Matrix verteilten Inseln basieren auf einem wasserlöslichen oder in Wasser quellbaren Material wie Polyvinylalkohol oder Polyvinylpyrrolidon.

Mit der EP 0 481 443 A1 werden transdermale therapeutische Zubereitungen offenbart, die eine verbesserte Lager-stabllität hinsichtlich der physikalisch-chemischen Struktur des Wirkstoffs aufweisen. Diese Zubereitungen umfassen eine steife Rückschicht, eine polymere Matrix und eine entfernbare Schutzschicht, wobei die polymere Matrix aus einem Polymerfilm besteht, in dem Partikel verteilt sind, die mit Wirkstoff und einer die Absorption des Wirkstoffs von der Haut verbessernden Verbindung beladen sind. Bei den Partikeln handelt es sich um mikroporöse Partikel oder polymere Mikrosphären, beispielsweise aus vernetztem Polyvinylpyrrolidon.

Die WO 01/01967 A1 offenbart transdermale therapeutische Systeme auf Basis von Polysiloxan, enthaltend Mikroreservoire mit Wirkstoff und einem ambiphilen Lösemittel. Als Beispiele für ambiphile Lösemittel werden 1,3-Butandiol, Dipropylenglykol, Tetrahydrofurfurylalkohol, Diethylenglykoldimethylether, Diethylenglykolmonoethylether, Diethylenglkyolmonobutylether, Propylenglykol, Dipropylenglykol, Carbonsäureester von Tri- und Diethylenglykol sowie polyoxyethylierte Fettalkohole von 6-18 C-Atomen genannt.

Die Offenlegungsschrift WO 01/68060 A2 beschreibt transdermale therapeutische Systeme vom Matrix-Typ mit einer wirkstoffundurchlässigen Rückschicht, einer ablösbaren Schutzschicht und einer wirkstoffhaltigen Matrix auf Basis von hydrophoben Polymeren, wobei der Wirkstoff einen Schmelzpunkt oberhalb der Raumtemperatur hat und zumindest während eines Teils der Anwendungsdauer des TTS in einer die Sättigungslöslichkeit überschreitenden Konzentration vorliegt.

Diese transdermalen therapeutischen Systeme zeichnen sich dadurch aus, dass den hydrophoben Basispolymeren der Wirkstoffmatrix ein Polyacrylatpolymer beigemischt ist, und/ oder die hydrophobe Polymere enthaltende Matrixschicht mit einer selbstklebenden Hautkontaktschicht auf der Basis von Polyacrylaten versehen ist. Zusätzlich zu den Polyacrylaten können auch Mischungen von Polyacrylaten mit anderen hydrophilen Polymeren wie Polyvinylpyrrolidon oder Copolymere des Vinylpyrrolidons mit Vinylacetat eingesetzt werden.

Mit der WO 95/18603 A1 werden transdermale Vorrichtungen offenbart, die Polyvinylpyrrolidon als Löslichkeitsverstärker enthalten. Eine Mischung aus mindestens drei Polymeren, beispielsweise einem oder mehreren Polysiloxanen, einem Polyacrylat und einem wasserlöslichen Polyvinylpyrrolidon, führen in Kombination mit dem Wirkstoff zu einer haftklebenden Zubereitung für ein transdermales therapeutisches System. Das lösliche Polyvinylpyrrolidon erhöht die Löslichkeit des Wirkstoffs, ohne sich negativ auf die Klebeigenschaften oder die Wirkstofffreigabe aus der haftklebenden Zubereitung auszuwirken.

In der WO 2011/076879 wird ein transdermales therapeutisches System beschrieben, das eine feste Dispersion aus dem Arzneistoff Rotigotin und Polyvinylpyrrolidon in einer Silikonhaftklebermischung umfasst, wobei Rotigotin und das Polyvinylpyrrolidon in einer Vielzahl von Mikroreservoiren vorliegen können. Transdermale therapeutische Systeme, die Mikroreservoire umfassen, sind ferner bereits aus WO 2004/012719 und WO 2004/012730 bekannt.

Trotz der zahlreichen Lösungsansätze ist das Problem nicht stabiler, übersättigter Systeme nicht grundsätzlich gelöst, denn es treten bei den auf dem Markt erhältlichen transdermalen therapeutischen Systemen immer wieder Formulierungen auf, bei denen der Arzneistoff kristallisiert.

Zudem sind noch längst nicht alle Arzneistoffe einer transdermalen Verabreichung erschlossen, weil es bislang nicht für jeden Arzneistoff gelungen ist, Systeme mit einer ausreichend hohen Arzneistoffkonzentration oder stabile übersättigte Systeme zu entwickeln.

Der vorliegenden Erfindung lag daher die Aufgabe zugrunde, ein transdermales therapeutisches System bereitzustellen, das hinsichtlich der stabilen Modifikation eines Arzneistoffs übersättigt, aber dennoch lagerstabil ist und keine spontane Rekristallisation des Arzneistoffs zeigt. Aufgrund der pharmazeutischen Erfahrungen mit zahlreichen Arzneistoffen wird der Anwendungskreis der vorliegenden Erfindung auf solche Arzneistoffe eingeschränkt, deren spontane Kristallisationsrate kinetisch verzögert eintritt.

Die Aufgabe wird gelöst durch das Bereitstellen eines transdermalen therapeutischen Systems (TTS), umfassend eine wirkstoffundurchlässige Rückschicht, eine Matrix bestehend aus einer, zwei oder mehr Schicht(en) und eine ablösbare Schutzschicht, dadurch gekennzeichnet, dass die Matrixschicht(en) oder mindestens eine der Matrixschichten mindestens einen Haftkleber, mindestens einen Arzneistoff sowie Partikel aus quervernetztem Polyvinylpyrrolidon umfassen/umfasst, wobei es sich bei dem Haftkleber um einen Schmelzhaftkleber handelt.

Grundsätzlich handelt es sich bei den transdermalen therapeutischen Systemen der vorliegenden Erfindung um Heißschmelz-TTS auf Basis von Schmelzhaftklebern, die lösungsmittelfrei hergestellt werden.

Ferner handelt es sich bei den erfindungsgemäßen transdermalen therapeutischen Systemen um Pflaster zur Applikation auf der Haut eines Patienten.

Die Begriffe "transdermales therapeutisches System", "TTS" und "Pflaster" werden im Zusammenhang mit der vorliegenden Erfindung synonym verwendet.

Der/die Arzneistoff(e) und die Partikel aus quervernetztem Polyvinylpyrrolidon bilden eine innere Phase und liegen in dem Haftkleber als äußerer Phase der erfindungsgemäßen transdermalen therapeutischen Systeme dispergiert vor. Der Haftkleber hat somit die Funktion eines Dispersionsmittels.

Als Schmelzhaftkleber im Sinne der vorliegenden Erfindung werden druckempfindliche heißschmelzfähige Haftkleber verstanden, die unter Druckanwendung eine Bindung mit einer nichtklebenden Fläche ausbilden und die unter Temperatureinfluss erweichen, so dass sie sich zu den erfindungsgemäßen transdermalen therapeutischen Systemen verarbeiten lassen. Heißschmelzfähige Haftkleber zur Verwendung in der vorliegenden Erfindung können sowohl aus einem Haftkleber als auch aus einer Mischung verschiedener Haftkleber bestehen.

Als Schmelzhaftkleber werden erfindungsgemäß solche bevorzugt, die aus Polydimethylsiloxan aufgebaute Silikonpolymere enthalten. Allgemein handelt es sich bei den erfindungsgemäß einsetzbaren Silikon-Haftklebern um auf Basis von Si-likonpolymeren hergestellte Schmelzhaftkleber, die vorzugsweise mindestens 50 Gew.-%, insbesondere 60-95 Gew.-%, besonders bevorzugt 75-90 Gew.-% Silikonpolymer(e), wie z.B. Silikonpolymere mit Polydimethylsiloxanstruktur oder Poly-dimethyldiphenylsiloxanstruktur, enthalten.

Gemäß einer bevorzugten Ausführungsform enthalten die erfindungsgemäßen transdermalen therapeutischen Systeme als Haftkleber mindestens einen der zuvor beschriebenen Silikon-Schmelzhaftkleber, der ferner bevorzugt ein aus Polydimethylsiloxan aufgebautes Silikonpolymer enthält.

Silikon-Schmelzhaftkleber zu Verwendung in der vorliegenden Erfindung können zusätzlich Silikonöle und/oder andere Weichmacher (Plasticizer) enthalten.

Des Weiteren kommen auch Mischungen oder Kondensate von Silikonharzen und Polyorganosiloxanen in Betracht.
Ferner werden aminresistente Silikon-Haftkleber bevorzugt, welche sich dadurch auszeichnen, dass sie keine oder nur wenige freie Silanolfunktionen enthalten, da die Si-OH-Gruppen alkyliert wurden.

Gemäß einer bevorzugten Ausführungsform wird ein Schmelzhaftkleber verwendet, der beim Erwärmen erweicht und dabei eine Viskosität erreicht, die sich für die Einarbeitung von einem, zwei oder mehr Arzneistoff/Arzneistoffen in fester Form und der Partikel aus quervernetztem PVP sowie ein Auftragen mittels Schlitzextrusion oder Beschichtung eignet, und der nach dem Abkühlen wieder in einem nicht fließfähigen Zustand vorliegt.

Die Erweichungstemperatur geeigneter Schmelzhaftkleber liegt im Bereich zwischen 50 und 200 °C, bevorzugt zwischen 75 und 170 °C und besonders bevorzugt zwischen 100 und 150 °C.

Bevorzugt weisen entsprechende Schmelzhaftkleber im erweichten Zustand eine dynamische Viskosität von höchstens 150 Pa·s, bevorzugt von höchstens 120 Pa·s, besonders bevorzugt von weniger als 100 Pa·s, ferner bevorzugt von weniger als 80 Pa·s und insbesondere bevorzugt von weniger als 60 Pa·s auf.

Ein geeigneter Silikon-Schmelzhaftkleber zur Verwendung in der vorliegenden Erfindung ist z.B. der Heißschmelzkleber BIO-PSA 7-4560 von Dow Corning.

Als Haftkleber kommen neben Silikon-Schmelzhaftklebern auch andere Schmelzhaftkleber in Betracht, sofern sie - wie die Silikon-Schmelzhaftkleber - eine hohe Wirkstoffdiffusibilität und eine geringe Wirkstofflöslichkeit aufweisen, wie z.B. Styrol-Block-Copolymer basierte heißschmelzfähige Kleber ("SXS-Haftkleber") oder Ethylenvinylacetat-Copolymer basierte heißschmelzfähige Kleber ("EVA-Haftkleber").

Vorzugsweise beträgt die Wirkstofflöslichkeit in den zur Verwendung in der vorliegenden Erfindung geeigneten Schmelzhaftklebern (d.h. die Löslichkeit eines Arzneistoffs in einem geeigneten Schmelzhaftkleber) 0-2 Gew.-% und bevorzugt 0-0,5 Gew.-%.

Schmelzhaftkleber und insbesondere Silikon-Schmelzhaftkleber, die zur Verwendung in der vorliegenden Erfindung geeignet sind und wie sie vorstehend beschrieben werden, sind dem Fachmann grundsätzlich bekannt und im Handel erhältlich.

Partikel aus quervernetztem Polyvinylpyrrolidon, d. h. quervernetztes Polyvinylpyrrolidon in partikulärer Form, sind bzw. ist dem Fachmann bekannt und im Handel erhältlich. Im Allgemeinen handelt es sich hierbei um Partikel mit einer mittleren Partikelgröße (Korngröße) von 5 µm bis 500 µm, wobei Partikel mit einer mittleren Partikelgröße von 5-100 µm bevorzugt sind. Sofern nicht vom Hersteller angegeben, kann die mittlere Partikelgröße auf dem Fachmann bekannte Weise ermittelt werden (z.B. durch eine Partikelgrößenbestimmung mittels Laserbeugung). Grundsätzlich sollten die Partikel aus quervernetztem Polyvinylpyrrolidon in ihrer längsten Ausdehnung jedoch nicht größer sein als die Dicke der Schicht bzw. der einzelnen Schichten der Matrix der erfindungsgemäßen transdermalen therapeutischen Systeme, die mindestens einen Arzneistoff und die Partikel aus quervernetztem Polyvinylpyrrolidon enthält/enthalten.

Quervernetztes Polyvinylpyrrolidon zeichnet sich dadurch aus, dass es in Wasser wie auch in organischen Lösemitteln unlöslich ist.

In den erfindungsgemäßen TTS sind die genannten Polyvinylpyrrolidon-Partikel in dem Schmelzhaftkleber, welcher die arzneistoffhaltige Matrixschicht(en) bildet, dispergiert.

Aufgrund der Wasserunlöslichkeit von quervernetztem Polyvinylpyrrolidon wird die Integrität der in den erfindungsgemäßen TTS dispergierten Polyvinylpyrrolidon-Partikel auch dann gewährleistet, wenn der Patient, während er eins, zwei oder mehr des/der erfindungsgemäßen TTS auf der Haut trägt, schwitzt bzw. zu schwitzen beginnt.

Die Gesamtmenge an quervernetztem Polyvinylpyrrolidon in den jeweiligen Schichten der Matrix der erfindungsgemäßen transdermalen therapeutischen Systeme, die mindestens einen Schmelzhaftkleber, mindestens einen Arzneistoff sowie Partikel aus quervernetztem Polyvinylpyrrolidon umfassen, beträgt 1-25 Gew.-%, vorzugsweise 2-15 Gew.-% und besonders bevorzugt 5-10 Gew.-%.

Gemäß einer bevorzugten Ausführungsform umfassen die arzneistoffhaltigen Matrixschichten der erfindungsgemäßen transdermalen therapeutischen Systeme mindestens einen Schmelzhaftkleber, insbesondere einen Silikon-Schmelzhaftkleber, mindestens einen Arzneistoff und Partikel aus quervernetztem Polyvinylpyrrolidon mit einer mittleren Partikelgröße von 5-500 µm, vorzugsweise 5-100 µm.

Der Arzneistoffgehalt der erfindungsgemäßen transdermalen therapeutischen Systeme beträgt vorzugsweise 5-25 Gew**.**-%, besonders bevorzugt 10-20 Gew.-% und insbesondere 15-20 Gew.-%, jeweils bezogen auf die arzneistoffhaltige(n) Matrixschicht(en) der erfindungsgemäßen transdermalen therapeutischen Systeme. Ferner wird erfindungsgemäß bevorzugt, dass die Arzneistoffkonzentration derart ist, dass die Matrixschicht(en) hinsichtlich der stabilen Modifikation des/der Arzneistoffs/Arzneistoffe übersättigt ist/sind.

Das Mengenverhältnis von Arzneistoff zu Polyvinylpyrrolidon in den erfindungsgemäßen transdermalen therapeutischen Systemen liegt vorzugsweise im Bereich von 10:1 bis 1:10.

Gemäß einer weiteren bevorzugten Ausführungsform umfasst/umfassen die arzneistoffhaltige(n) Matrixschicht(en) der erfindungsgemäßen transdermalen therapeutischen Systeme mindestens einen Schmelzhaftkleber, insbesondere einen Silikon-Schmelzhaftkleber, mindestens einen Arzneistoff und Partikel aus quervernetztem Polyvinylpyrrolidon mit einer mittleren Partikelgröße von 5-500 µm, vorzugsweise 5-100 µm, wobei das Mengenverhältnis des/der Arzneistoffs/der Arzneistoffe zu Polyvinylpyrrolidon im Bereich von 10:1 bis 1:10 liegt.

Als Arzneistoffe für die erfindungsgemäßen transdermalen therapeutischen Systeme kommen pharmazeutische Wirkstoffe (sowie deren Salze) in Betracht, vorzugsweise solche, die eine geringe Neigung zur spontanen Kristallisation aufweisen. Besonders geeignet sind die Arzneistoffe, die aus der Gruppe von pharmazeutischen Wirkstoffen ausgewählt sind, die Estradiol, vorzugsweise wasserfreies Estradiol, sowie Buprenorphin, Rotigotin, Rivastigmin, Scopolamin, Granisetron, Lerisetron, Ramosetron, Ondansetron und Pramipexol, sowie pharmazeutisch akzeptable Salze der vorgenannten Stoffe umfasst.

Gemäß einer bevorzugten Ausführungsform werden wasserfreies Estradiol, Scopolamin oder Rotigotin als Arzneistoff in den erfindungsgemäßen transdermalen therapeutischen Systemen verwendet.

Die jeweiligen Arzneistoffe liegen in den fertigen erfindungsgemäßen transdermalen therapeutischen Systemen in einer nicht kristallinen Form vor.

Der Begriff "nicht kristalline Form" bedeutet im Zusammenhang der vorliegenden Erfindung, dass der jeweilige Arzneistoff sowohl in Form einer festen Lösung als auch in amorpher Form sowie in beiden Formen nebeneinander vorliegen kann.

Es wird davon ausgegangen, ohne durch diese Theorie gebunden zu sein, dass der/die Arzneistoff(e) in dem Dispersionsmittel/der äußeren Phase, d.h. dem Haftkleber, molekular dispers vorliegt/vorliegen und dass eine nicht kristalline Form des Arzneistoffs/der Arzneistoffe reversibel mit den Partikeln aus quervernetztem PVP verbunden ist, wobei der/die mit den Partikeln aus quervernetztem PVP verbundene (n) Arzneistoff(e) eine innere Phase in Form einer Vielzahl von Mikroreservoiren bilden.

Dies schließt nicht aus und wird normalerweise sogar implizieren, dass ein bestimmter Anteil des Arzneistoffs/der Arzneistoffe in dem Dispersionsmittel bis zu seiner Sättigungskonzentration gelöst vorliegt.

Im Rahmen dieser Beschreibung sollen "Mikroreservoire" als partikuläre, räumlich und funktionell getrennte Kompartimente verstanden werden, die aus einer Mischung von Arzneistoff und quervernetztem PVP bestehen und die in dem Haftkleber der erfindungsgemäßen transdermalen therapeutischen Systeme dispergiert sind. Bevorzugt enthalten die erfindungsgemäßen transdermalen therapeutischen Systeme 10³ bis 10⁹ und besonders bevorzugt 10⁶ bis 10⁹ Mikroreservoire pro cm² ihrer Oberfläche.

Der maximale Durchmesser der Mikroreservoire ist kleiner als die Dicke der arzneistoffhaltigen Matrixschicht(en) der erfindungsgemäßen transdermalen therapeutischen Systeme und beträgt bevorzugt bis zu 70 % der Dicke der Matrixschicht(en) und besonders bevorzugt 5 bis 60 % der Dicke der Matrixschicht(en). Für eine beispielhafte Dicke der Matrixschicht (en) von 50 µm entspricht dies einem maximalen Durchmesser der Mikroreservoire im Bereich von bevorzugt bis zu 35 µm.

Der Begriff "maximaler Durchmesser" bezeichnet den Durchmesser der Mikroreservoire der innerhalb der drei Raumdimensionen (x-, y- oder z-Dimension) der größte ist. Es ist für den Fachmann klar, dass im Fall von sphärischen Mikroresvoiren der maximale Durchmesser dem Durchmesser der Mikroreservoire entspricht. Im Fall von Mikroreservoiren, die nicht kugelförmig ausgebildet sind, d.h. in unterschiedlichen geometrischen Formen vorliegen, kann sich deren Ausdehnung in den jeweiligen x-, y- und z-Dimensionen stark unterscheiden.

Gemäß einer besonders bevorzugten Ausführungsform liegt der mittlere Durchmesser der Mikroreservoire, die in der/den arzneistoffhaltigen Matrixschicht(en) der erfindungsgemäßen transdermalen therapeutischen Systeme verteilt sind, im Bereich von 1 bis 40 %, stärker bevorzugt im Bereich von 1 bis 20 %, der Dicke der Matrixschicht(en). Für eine beispielhafte Dicke der Matrixschicht(en) von 50 µm entspricht dies einem mittleren Durchmesser der Mikroreservoire im Bereich von bevorzugt 0,5 bis 20 µm.

Der Begriff "mittlerer Durchmesser" bezeichnet den Mittelwert des x-, y- und z-Durchschnittsdurchmessers aller Mikroreservoire.

Die maximalen und mittleren Durchmesser der Mikroreservoire sowie die Anzahl der Mikroreservoire pro Oberfläche können wie folgt bestimmt werden. Nach Entfernung der ablösbaren Schutzschicht wird die Oberfläche der jeweiligen transdermalen therapeutischen Systeme mit einem Lichtmikroskop untersucht (z.B. mit einem Leica Mikroskop Typ DM/RBE, ausgestattet mit einer Kamera Typ Basler A 113C). Die Messung wird durch die zufällige Analyse mit polarisiertem Licht unter Verwendung eines Mikroskops bei 200-facher Vergrößerung durchgeführt. Eine Bildanalyse kann z.B. unter Verwendung der Software Nikon LuciaDi, Version 4.21, durchgeführt werden, was zu mittleren und maximalen Durchmessern für jede Probe führt.

In einer bevorzugten Ausführungsform enthalten die erfindungsgemäßen transdermalen therapeutischen Systeme einen Arzneistoff oder zwei Arzneistoffe und besonders bevorzugt mindestens einen Arzneistoff. In einer weiteren Ausführungsform können die erfindungsgemäßen transdermalen therapeutischen Systeme auch zwei oder mehr Arzneistoffe enthalten.

Im Hinblick auf die Lösung der Aufgabe, die der vorliegenden Erfindung zugrunde lag, sollte das Auftreten von Kristallisationskeimen während des Herstellungsprozesses möglichst vermieden werden. Daher erfolgt die Herstellung der erfindungsgemäßen transdermalen therapeutischen Systeme bevorzugt unter Anwendung von Wärme.

Dies wird erfindungsgemäß dadurch bewerkstelligt, dass die Temperatur der arzneistoffhaltigen Schmelzhaftklebermasse oder der daraus hergestellten arzneistoffhaltigen haftklebenden Matrixschicht(en) der erfindungsgemäßen transdermalen therapeutischen Systeme bzw. Pflaster während des Herstellungsprozesses zumindest zeitweise über dem Schmelzpunkt oder dem Schmelzbereich der stabilen Modifikation des/der einzuarbeitenden Arzneistoffs/Arzneistoffe liegt. Diese Temperaturbehandlung kann während der Herstellung der arzneistoffhaltigen Schmelzhaftklebermasse und/oder der Beschichtung der wirkstoffundurchlässigen Rückschicht vorgenommen werden.

Vorzugsweise liegt die erwähnte Temperatur mindestens 5-10 °C, besonders bevorzugt mindestens 10 °C und insbesondere 5 bis 50 °C und bevorzugt 10 bis 25 °C über dem Schmelzpunkt oder dem Schmelzbereich der stabilen Modifikation des jeweiligen Arzneistoffs, wobei der Bezugspunkt im Fall des Schmelzbereichs im Rahmen der vorliegenden Erfindung dessen Maximalwert ist.

Der Begriff "stabile Modifikation des jeweiligen Arzneistoffs" bezeichnet insbesondere die thermodynamisch stabile Modifikation des jeweiligen Arzneistoffs. Ferner umfasst der Begriff "stabile Modifikation des jeweiligen Arzneistoffs" im Rahmen der vorliegenden Erfindung sowohl die kristalline Form, einschließlich verschiedener Kristallmodifikationen (soweit vorhanden), also auch die amorphe Form eines Arzneistoffs.

Für die vorstehend beschriebene Temperaturbehandlung reicht es aus, wenn die genannte Temperatur kurzzeitig erreicht bzw. beibehalten wird, vorzugsweise für eine Dauer von mindestens 1 min, insbesondere von 1 min bis 10 min.

Die stabilen Modifikationen der jeweiligen Arzneistoffe sowie die Schmelzpunkte oder Schmelzbereiche dieser stabilen Modifikationen sind dem Fachmann bekannt oder können der Fachliteratur entnommen werden.

Nachfolgend sind beispielhaft die Schmelzpunkte bzw. Schmelzbereiche der stabilen Modifikationen einiger erfindungsgemäß einsetzbarer Arzneistoffe aufgeführt:
Estradiol: 173-179 °C;
Buprenorphin: 209 °C;
Rotigotin: 97 °C;
Rivastigmin-Tartrat: 123-125 °C;
Scopolamin-Hydrobromid: 195 °C;
Granisetron-Hydrochlorid: 290-292 °C;
Ondansetron-Hydrochlorid: 178,5-179,5 °C;
Pramipexol-Dihydrochlorid: 296-298 °C.

Der erfindungsgemäße Erfolg, nämlich die Vermeidung der Rekristallisation des Arzneistoffs in einer mit Arzneistoff übersättigten Matrixschicht eines transdermalen therapeutischen Systems, tritt insbesondere bei der Kombination der erfindungsgemäßen Formulierungen mit der vorstehend beschriebenen Temperaturbehandlung ein.

Für die Anwendung des erfindungsgemäßen Herstellungsverfahrens sind insbesondere solche Arzneistoffe geeignet, die einen Schmelzpunkt oder Schmelzbereich haben, der zwischen 20°C und 350°C liegt. Besonders geeignet sind Arzneistoffe mit einem Schmelzpunkt oder einem Schmelzbereich zwischen 30°C und 200 °C.

Ob ein bestimmter Arzneistoff eine geringe Neigung zur spontanen Kristallisation aufweist und somit für die vorliegende Erfindung besonders geeignet ist, lässt sich durch ein Vorexperiment ermitteln, in dem zunächst die Sättigungslöslichkeit des Arzneistoffs in einem Lösemittel, vorzugsweise Ethanol, ermittelt wird.

Dazu wird eine Suspension der stabilen Modifikation des Arzneistoffs in dem Lösemittel bei 25°C über 24 Stunden gerührt, so dass sich ein Gleichgewicht einstellt. Nach einer Filtration wird der Arzneistoffgehalt im Überstand durch eine geeignete analytische Methode bestimmt, die dem Fachmann bekannt ist. Anschließend wird mit dem gleichen Lösemittel eine doppelt konzentrierte Lösung hergestellt, indem eine entsprechende Menge Arzneistoff in das Lösemittel eingewogen wird. Die zunächst entstehende Suspension wird solange erwärmt, bis sich alle Reste des kristallinen Arzneistoffs in dem Lösemittel gelöst haben. Während dieser Zeit verdampftes Lösemittel ist zu ergänzen und die Lösung sodann abzukühlen.

Eine Glasampulle mit einem Volumen von maximal 1 ml wird mit der Lösung befüllt und die Lösung in der verschlossenen Ampulle noch einmal für mindestens 10 Minuten auf eine Temperatur erwärmt, die mindestens 10°C über dem Schmelzpunkt (bzw. über dem Schmelzbereich) der stabilen Modifikation des Arzneistoffs liegt, und dann für 24 Stunden bei 25°C gelagert. Arzneistoffe, die unter diesen Bedingungen in dem flüssigen Lösemittel gelöst bleiben, zeigen eine geringe Neigung zur spontanen Kristallisation und sind somit besonders geeignet, um mit dem erfindungsgemäßen Verfahren zu einem erfindungsgemäßen TTS verarbeitet werden zu können.

Zur Herstellung der erfindungsgemäßen transdermalen therapeutischen Systeme wird ein Verfahren bevorzugt, bei dem die arzneistoffhaltige Schmelzhaftklebermasse für die Matrixschicht(en) lösemittelfrei erzeugt wird. Dafür wird der Schmelzhaftkleber soweit erwärmt, dass festes, in Form von Partikeln vorliegendes, quervernetztes Polyvinylpyrrolidon und die erforderliche Menge von einem, zwei oder mehr und bevorzugt von mindestens einem kristallinen oder amorphen Arzneistoff/Arzneistoffen zugefügt und durch Verkneten oder Verrühren in der Schmelzhaftklebermasse dispergiert werden können. Vorzugsweise wird als Schmelzhaftkleber ein Silikon-Schmelzhaftkleber verwendet.

Der Schmelzhaftkleber wird dabei auf eine Temperatur erhitzt, die 5-20 °C und bevorzugt ca. 10 °C über der Erweichungstemperatur der Schmelzhaftklebermasse und gleichzeitig unterhalb des Schmelzpunkts oder des Schmelzbereichs der stabilen Modifikation des/der einzuarbeitenden Arzneistoffs/Arzneistoffe liegt.

Die Auswahl eines der zuvor beschriebenen Schmelzhaftklebers mit einem entsprechenden Schmelzbereich für die Verwendung in den erfindungsgemäßen transdermalen therapeutischen Systemen richtet sich somit auch nach dem/den einzuarbeitenden Arzneistoff/Arzneistoffen und ihren jeweiligen Schmelzpunkten oder Schmelzbereichen.

Zusätzlich können der Schmelzhaftklebermasse pharmazeutische Hilfsstoffe, die dem Fachmann bekannt sind, beispielsweise Permeationsverstärker, Weichmacher, Antioxidantien und dergleichen, zugesetzt werden.

Die so erhaltene arzneistoffhaltige Schmelzhaftklebermasse wird durch ein geeignetes Verfahren, beispielsweise durch Schlitzextrusion oder Beschichtung mit einer Rakelwalze oder einem Streichkasten, auf einen geeigneten film- oder folienförmigen Polymerträger aufgebracht. In einer bevorzugten Ausführungsform bildet der film- oder folienförmige Polymerträger die wirkstoffundurchlässige Rückschicht des fertigen TTS.

Unmittelbar nach der Beschichtung des Polymerträgers kühlt die aufgetragene Schmelzhaftklebermasse ab und kann, da spontan klebrig, mit einem weiteren Polymerfilm oder einer weiteren Polymerfolie abgedeckt werden. Es ist aber auch möglich, weitere arzneistoffhaltige Beschichtungen auf den bereits beschichteten Polymerträger aufzubringen, bevor mit dem weiteren Polymerfilm oder der weiteren Polymerfolie abgedeckt wird.

Gemäß einer bevorzugten Ausführungsform bildet der weitere Polymerfilm oder die weitere Polymerfolie die ablösbare Schutzschicht des fertigen TTS.

Durch Ausstanzen aus dem erhaltenen Laminat, das aus einer, zwei oder mehr arzneistoffhaltigen Matrixschicht(en) zwischen zwei Lagen Polymerfolie oder Polymerfilm besteht, entstehen die einzelnen transdermalen therapeutischen Systeme der vorliegenden Erfindung, die in Einzelverpackungen verpackt werden können.

Während der Herstellung der erfindungsgemäßen transdermalen therapeutischen Systeme gemäß dem zuvor beschriebenen Verfahren wird zusätzlich eine Temperaturbehandlung durchgeführt, bei der die Temperatur während der Herstellung der arzneistoffhaltigen Schmelzhaftklebermasse und/oder der Beschichtung des Polymerträgers zumindest zeitweise über den Schmelzpunkt oder den Schmelzbereich der stabilen Modifikation des/der jeweiligen Arzneistoffs/Arzneistoffe erhöht wird.

Bevorzugt erfolgt die Temperaturbehandlung in dem zuvor beschriebenen Verfahren bei einer Temperatur, die mindestens 5-10 °C, besonders bevorzugt mindestens 10 °C, und insbesondere 5 bis 50 °C und bevorzugt 10 bis 25 °C, über dem Schmelzpunkt oder dem Schmelzbereich der stabilen Modifikation des/der jeweiligen Arzneistoffs/Arzneistoffe liegt.

Für die Temperaturbehandlung reicht es aus, wenn die genannte Temperatur kurzzeitig erreicht bzw. beibehalten wird, vorzugsweise für eine Dauer von mindestens 1 min, insbesondere von 1 min bis 10 min.

Bevorzugt ist auch ein transdermales therapeutisches System gemäß dem Vorstehenden, dass dadurch gekennzeichnet ist, dass während der Herstellung der arzneistoffhaltigen Matrixschicht(en) und/oder der Beschichtung der wirkstoffundurchlässige Rückschicht des transdermalen therapeutischen Systems eine Temperaturbehandlung durchgeführt wurde, bei der die Temperatur über dem Schmelzpunkt oder dem Schmelzbereich, vorzugsweise mindestens 5-10 °C, besonders bevorzugt mindestens 10 °C, und insbesondere 5 bis 50 °C und bevorzugt 10 bis 25 °C über dem Schmelzpunkt oder dem Schmelzbereich, der stabilen Modifikation des/der Arzneistoffs/Arzneistoffe lag.

In einer bevorzugten Ausführungsform umfasst das Verfahren zur Herstellung der erfindungsgemäßen transdermalen therapeutischen Systeme
a) das Herstellen einer arzneistoffhaltigen Haftklebermasse, umfassend mindestens einen Haftkleber, mindestens einen Arzneistoff und Partikel aus quervernetztem Polyvinylpyrrolidon,
   wobei es sich bei dem Haftkleber um einen Schmelzhaftkleber handelt und der Arzneistoff und die Polyvinylpyrrolidon-Partikel in dem Schmelzhaftkleber dispergiert werden;
b) das Beschichten eines film- oder folienförmigen Polymerträgers mit der arzneistoffhaltigen Haftklebermasse;
und ist dadurch gekennzeichnet, dass während der Herstellung eine Temperaturbehandlung erfolgt, wobei die Temperatur über dem Schmelzpunkt oder dem Schmelzbereich der stabilen Modifikation des/der Arzneistoffs/Arzneistoffe liegt.

Bevorzugt liegt die Temperatur während der Temperaturbehandlung mindestens 10°C über dem Schmelzpunkt oder dem Schmelzbereich der stabilen Modifikation des/der Arzneistoffs/Arzneistoffe.

In einer weiteren bevorzugten Ausführungsform umfasst das Verfahren die folgenden zusätzlichen Schritte:
c) Abdecken der Beschichtung mit einer Polymerfolie oder einem Polymerfilm,
d) Ausstanzen von einzelnen transdermalen therapeutischen Systemen,
e) Verpacken der einzelnen transdermalen therapeutischen Systeme.

Besonders bevorzugt erfolgt die Temperaturbehandlung in dem zuvor beschriebenen Verfahren während der Herstellung der arzneistoffhaltigen Haftklebermasse und/oder der Beschichtung des film- oder folienförmigen Polymerträgers.

In einer besonders bevorzugten Ausführungsform handelt es sich bei dem in dem zuvor beschriebenen Verfahren verwendeten Schmelzhaftkleber um einen Silikon-Schmelzhaftkleber, der ferner bevorzugt ein aus Polydimethylsiloxan aufgebautes Silikonpolymer enthält.

Bevorzugt ist ferner ein transdermales therapeutisches System, das mittels des zuvor beschriebenen Verfahrens hergestellt wurde.

### Beispiel:

Eine heißschmelzfähige Klebstoffmasse (Schmelzhaftklebermasse) auf Basis eines Silikonpolymers wird auf eine Temperatur erhitzt, die 10 °C über der Erweichungstemperatur des Schmelzhaftklebers liegt. Dann werden 15 Gew.-% quervernetztes Polyvinylpyrrolidon mit einer mittleren Partikelgröße von 20 µm und eventuell erforderliche Stabilisatoren hinzugefügt. Durch Kneten werden die Polyvinylpyrrolidon-Partikel in der Schmelzhaftklebermasse dispergiert. Anschließend werden 10 Gew.-% eines Arzneistoffs in fester Form hinzugefügt und ebenfalls durch Kneten in der Schmelzhaftklebermasse verteilt. Schließlich wird die Schmelzhaftklebermasse auf eine Temperatur erhitzt, welche 10 °C über der Schmelztemperatur oder dem Schmelzbereich der stabilen Modifikation des Arzneistoffs liegt.

Die so erhaltene arzneistoffhaltige Schmelzhaftklebermasse wird durch eine Schlitzdüse in einer Dicke von 100 µm auf einen folienförmigen Polymerträger in Form einer Polyesterfolie von 20 µm Dicke extrudiert und die extrudierte Schicht mit einer geeigneten Schutzfolie abgedeckt. Aus dem so erhaltenen Gesamtlaminat werden nun einzelne TTS ausgestanzt und in Siegelbeutel verpackt.

## Patentansprüche

1. Transdermales therapeutisches System (TTS), umfassend eine wirkstoffundurchlässige Rückschicht, eine Matrix bestehend aus einer, zwei oder mehr Schicht(en) und eine ablösbare Schutzschicht, **dadurch gekennzeichnet,**
**dass** die Matrixschicht(en) oder mindestens eine der Matrixschichten mindestens einen Haftkleber, mindestens einen Arzneistoff sowie Partikel aus quervernetztem Polyvinylpyrrolidon umfassen/umfasst und
**dass** während der Herstellung der arzneistoffhaltigen Matrixschicht(en) und/oder der Beschichtung der wirkstoffundurchlässige Rückschicht des transdermalen therapeutischen Systems eine Temperaturbehandlung durchgeführt wurde, bei der die Temperatur über dem Schmelzpunkt oder dem Schmelzbereich, vorzugsweise mindestens 10 °C über dem Schmelzpunkt oder dem Schmelzbereich, der stabilen Modifikation des/der Arzneistoffs/Arzneistoffe lag,
wobei es sich bei dem Haftkleber um einen Schmelzhaftkleber handelt.

2. Transdermales therapeutisches System nach Anspruch 1, **dadurch gekennzeichnet, dass** der Schmelzhaftkleber ein Silikon-Schmelzhaftkleber ist, der ein aus Polydimethylsiloxan aufgebautes Silikonpolymer enthält.

3. Transdermales therapeutisches System nach einem der Ansprüche 1 bis 2, **dadurch gekennzeichnet, dass** die mittlere Partikelgröße der Partikel aus quervernetztem Polyvinylpyrrolidon 5-500 µm, vorzugsweise 5-100 µm beträgt.

4. Transdermales therapeutisches System nach einem der vorangehenden Ansprüche, **dadurch gekennzeichnet, dass** der/die Arzneistoff(e) eine geringe Neigung zur spontanen Kristallisation aufweist.

5. Transdermales therapeutisches System nach einem der vorangehenden Ansprüche, **dadurch gekennzeichnet, dass** der/die Arzneistoff(e) aus der Gruppe ausgewählt ist/sind, die wasserfreies Estradiol sowie Buprenorphin, Rotigotin, Rivastigmin, Scopolamin, Granisetron, Lerisetron, Ramosetron, Ondansetron und Pramipexol und pharmazeutisch akzeptable Salze der vorgenannten Arzneistoffe umfasst.

6. Transdermales therapeutisches System nach einem der vorangehenden Ansprüche, **dadurch gekennzeichnet, dass** das Mengenverhältnis des/der Arzneistoffs/Arzneistoffe zu Polyvinylpyrrolidon im Bereich von 10:1 bis 1:10 liegt.

7. Transdermales therapeutisches System nach einem der vorangehenden Ansprüche, **dadurch gekennzeichnet, dass** der oder die Arzneistoff(e) sowie die Partikel aus quervernetztem Polyvinylpyrrolidon in einer Vielzahl von Mikroreservoiren vorliegen.

8. Verfahren zur Herstellung eines TTS, umfassend
a) das Herstellen einer arzneistoffhaltigen Haftklebermasse, umfassend mindestens einen Haftkleber, mindestens einen Arzneistoff und Partikel aus quervernetztem Polyvinylpyrrolidon,
wobei es sich bei dem Haftkleber um einen Schmelzhaftkleber handelt und der Arzneistoff und die Polyvinylpyrrolidon-Partikel in dem Schmelzhaftkleber dispergiert werden;
b) das Beschichten eines film- oder folienförmigen Polymerträgers mit der arzneistoffhaltigen Haftklebermasse;
**dadurch gekennzeichnet, dass** während der Herstellung eine Temperaturbehandlung erfolgt, wobei die Temperatur über dem Schmelzpunkt oder dem Schmelzbereich der stabilen Modifikation des/der Arzneistoffs/Arzneistoffe liegt.

9. Verfahren nach Anspruch 8, **dadurch gekennzeichnet, dass** die Temperatur während der Temperaturbehandlung mindestens 10°C über dem Schmelzpunkt oder dem Schmelzbereich der stabilen Modifikation des/der Arzneistoffs/Arzneistoffe liegt.

10. Verfahren nach einem der Ansprüche 8 oder 9, **gekennzeichnet durch** die folgenden zusätzlichen Schritte:
c) Abdecken der Beschichtung mit einer Polymerfolie oder einem Polymerfilm,
d) Ausstanzen von einzelnen transdermalen therapeutischen Systemen,
e) Verpacken der einzelnen transdermalen therapeutischen Systeme.

11. Verfahren nach einem der Ansprüche 8 bis 10, **dadurch gekennzeichnet, dass** die Temperaturbehandlung während der Herstellung der arzneistoffhaltigen Haftklebermasse und/oder der Beschichtung des film- oder folienförmigen Polymerträgers erfolgt.

12. Verfahren nach einem der Ansprüche 8 bis 11, **dadurch gekennzeichnet, dass** es sich bei dem Schmelzhaftkleber um einen Silikon-Schmelzhaftkleber handelt, der ein aus Polydimethylsiloxan aufgebautes Silikonpolymer enthält.

13. Transdermales therapeutisches System, umfassend eine wirkstoffundurchlässige Rückschicht, eine Matrix bestehend aus einer, zwei oder mehr Schicht(en) und eine ablösbare Schutzschicht, **dadurch gekennzeichnet, dass** die Matrixschicht(en) oder mindestens eine der Matrixschichten mindestens einen Haftkleber, mindestens einen Arzneistoff sowie Partikel aus quervernetztem Polyvinylpyrrolidon umfassen/umfasst, wobei es sich bei dem Haftkleber um einen Schmelzhaftkleber handelt, hergestellt mittels eines Verfahrens nach einem der Ansprüche 8-12.

## Claims

1. Transdermal therapeutic system (TTS) comprising a backing layer, which is impermeable to active ingredient, a matrix consisting of one, two or more layer(s) and a detachable protective layer, **characterised in that** the matrix layer(s) or at least one of the matrix layers comprise/comprises at least one pressure-sensitive adhesive, at least one active pharmaceutical ingredient and particles of crosslinked polyvinylpyrrolidone and **in that** during the production of the active pharmaceutical ingredient-containing matrix layer(s) and/or the coating of the backing layer, which is impermeable to active ingredient, of the transdermal therapeutic system, heat treatment has been carried out in which the temperature was above the melting point or the melting range, preferably at least 10 °C above the melting point or the melting range, of the stable modification of the active pharmaceutical ingredient/active pharmaceutical ingredients, wherein the pressure-sensitive adhesive is a hot-melt pressure-sensitive adhesive.

2. Transdermal therapeutic system according to claim 1, **characterised in that** the hot-melt pressure-sensitive adhesive is a silicone hot-melt pressure-sensitive adhesive which contains a silicone polymer synthesised from polydimethylsiloxane.

3. Transdermal therapeutic system according to one of claims 1 to 2, **characterised in that** the average particle size of the particles of crosslinked polyvinylpyrrolidone is 5-500 µm, preferably 5-100 µm.

4. Transdermal therapeutic system according to one of the preceding claims, **characterised in that** the active pharmaceutical ingredient(s) has/have a slight tendency for spontaneous crystallisation.

5. Transdermal therapeutic system according to one of the preceding claims, **characterised in that** the active pharmaceutical ingredient(s) is/are selected from the group which comprises anhydrous estradiol as well as buprenorphine, rotigotine, rivastigmine, scopolamine, granisetron, lerisetron, ramosetron, ondansetron and pramipexole and pharmaceutically acceptable salts of the aforementioned active pharmaceutical ingredients.

6. Transdermal therapeutic system according to one of the preceding claims, **characterised in that** the proportion of the active pharmaceutical ingredient/active pharmaceutical ingredients to polyvinylpyrrolidone lies in the range from 10:1 to 1:10.

7. Transdermal therapeutic system according to one of the preceding claims, **characterised in that** the active pharmaceutical ingredient(s) and the particles of crosslinked polyvinylpyrrolidone are present in a plurality of micro-reservoirs.

8. Method for producing a TTS comprising
a) the production of an active pharmaceutical ingredient-containing pressure-sensitive adhesive mass, comprising at least one pressure-sensitive adhesive, at least one active pharmaceutical ingredient and particles of crosslinked polyvinylpyrrolidone, wherein the pressure-sensitive adhesive is a hot-melt pressure-sensitive adhesive and the active pharmaceutical ingredient and the polyvinylpyrrolidone particles are dispersed in the hot-melt pressure-sensitive adhesive;
b) the coating of a film-like or sheet-like polymer support with the active pharmaceutical ingredient-containing pressure-sensitive adhesive mass;
**characterised in that** during production, a heat treatment is effected, wherein the temperature is above the melting point or the melting range of the stable modification of the active pharmaceutical ingredient/active pharmaceutical ingredients.

9. Method according to claim 8, **characterised in that** the temperature during the heat treatment is at least 10 °C above the melting point or the melting range of the stable modification of the active pharmaceutical ingredient/active pharmaceutical ingredients.

10. Method according to one of claims 8 or 9, **characterised by** the following additional steps:
c) covering the coating with a polymer sheet or a polymer film,
d) punching out individual transdermal therapeutic systems,
e) packaging the individual transdermal therapeutic systems.

11. Method according to one of claims 8 to 10, **characterised in that** the heat treatment is effected during the production of the active pharmaceutical ingredient-containing pressure-sensitive adhesive mass and/or the coating of the film-like or sheet-like polymer support.

12. Method according to one of claims 8 to 11, **characterised in that** the hot-melt pressure-sensitive adhesive is a silicone hot-melt pressure-sensitive adhesive which contains a silicone polymer synthesised from polydimethylsiloxane.

13. Transdermal therapeutic system comprising a backing layer, which is impermeable to active ingredient, a matrix consisting of one, two or more layer(s) and a detachable protective layer, **characterised in that** the matrix layer(s) or at least one of the matrix layers comprise/comprises at least one pressure-sensitive adhesive, at least one active pharmaceutical ingredient as well as particles of crosslinked polyvinylpyrrolidone, wherein the pressure-sensitive adhesive is a hot-melt pressure-sensitive adhesive, produced by means of a method according to one of claims 8-12.

## Revendications

1. Système thérapeutique transdermique (TTS), comprenant une couche arrière imperméable aux substances actives, une matrice constituée d'une, de deux ou de plus de couche(s), et une couche protectrice amovible, **caractérisé :**
**en ce que** la ou les couche(s) de la matrice ou au moins l'une des couches de la matrice comprend (comprennent) au moins un auto-adhésif, au moins une substance active de médicament, ainsi que des particules de polyvinylpyrrolidone réticulée, et
**en ce que**, pendant la production de la (des) couche(s) contenant une substance active de médicament et/ou le revêtement de la couche arrière imperméable aux substances actives du système thérapeutique transdermique, on a effectué un traitement thermique dans lequel la température se situait au-dessus du point de fusion ou de la plage de fusion de préférence au moins 10 °C au-dessus du point de fusion ou de la plage de fusion, de la modification stable de la (des) substance(s) active(s) de médicament,
dans lequel, en ce qui concerne l'auto-adhésif, il s'agit d'un auto-adhésif thermofusible.

2. Système thérapeutique transdermique selon la revendication 1, **caractérisé en ce que** l'auto-adhésif thermofusible est un auto-adhésif thermofusible à base de silicone, qui contient un polymère de silicone constitué de polydiméthylsiloxane.

3. Système thérapeutique transdermique selon une des revendications 1 à 2, **caractérisé en ce que** la taille moyenne de particule des particules de polyvinylpyrrolidone réticulée est de 5 à 500 µm, de préférence de 5 à 100 µm.

4. Système thérapeutique transdermique selon une des revendications précédentes, **caractérisé en ce que** la(les) substance(s) active(s) de médicament présente(nt) une tendance réduite à la cristallisation spontanée.

5. Système thérapeutique transdermique selon une des revendications précédentes, **caractérisé en ce que** la(les) substance(s) active(s) de médicament est(sont) choisie(s) dans le groupe qui comprend l'oestradiol anhydre, ainsi que la buprénorphine, la rotigotine, la rivastigmine, la scopolamine, le granisétron, le lérisétron, le ramosétron, l'ondansétron et le pramipexole, et des sels pharmaceutiquement acceptables des substances actives énumérées ci-dessus.

6. Système thérapeutique transdermique selon une des revendications précédentes, **caractérisé en ce que** le rapport quantitatif de la (des) substance(s) active(s) à la polyvinylpyrrolidone se situe dans la plage de 10:1 à 1:10.

7. Système thérapeutique transdermique selon une des revendications précédentes, **caractérisé en ce que** la ou les substance(s) active(s) ainsi que les particules de polyvinylpyrrolidone réticulée sont présentes dans une pluralité de microréservoirs.

8. Procédé pour la production d'un TTS, comprenant :
a) la production d'une masse d'auto-adhésif qui contient une substance active de médicament, comprenant au moins un auto-adhésif, au moins une substance active de médicament et des particules de polyvinylpyrrolidone réticulée,
dans laquelle, en ce qui concerne l'auto-adhésif, il s'agit d'un auto-adhésif thermofusible, et la substance active de médicament et les particules de polyvinylpyrrolidone sont dispersées dans l'auto-adhésif thermofusible ;
b) le revêtement d'un support de polymère en forme de film ou de feuille avec la masse d'auto-adhésif qui contient une substance active de médicament ;
**caractérisé en ce que**, pendant la production, on effectue un traitement thermique dans lequel la température se situe au-dessus du point de fusion ou de la plage de fusion de la modification stable de la (des) substance(s) active(s) de médicament.

9. Procédé selon la revendication 8, **caractérisé en ce que** la température se situe, pendant le traitement thermique, au moins 10 °C au-dessus du point de fusion ou de la plage de fusion de la modification stable de la (des) substance(s) active(s) de médicament.

10. Procédé selon une des revendications 8 ou 9, **caractérisé par** les étapes supplémentaires suivantes :
c) recouvrement du revêtement avec une feuille de polymère ou un film de polymère,
d) découpage de systèmes thérapeutique transdermique individuels,
e) conditionnement des systèmes thérapeutique transdermique individuels.

11. Procédé selon une des revendications 8 à 10, **caractérisé en ce que** le traitement thermique a lieu pendant la production de la masse d'auto-adhésif qui contient une substance active de médicament et/ou pendant le revêtement du support de polymère en forme de film ou de feuille.

12. Procédé selon une des revendications 8 à 11, **caractérisé en ce que**, en ce qui concerne l'auto-adhésif thermofusible, il s'agit d'un auto-adhésif thermofusible à base de silicone, qui contient un polymère de silicone constitué de polydiméthylsiloxane.

13. Système thérapeutique transdermique, comprenant une couche arrière imperméable aux substances actives, une matrice constituée d'une, de deux ou de plus de couche(s), et une couche protectrice amovible, **caractérisé en ce que** la(les) couche(s) de la matrice ou au moins l'une des couches de la matrice comprend (comprennent) au moins un auto-adhésif, au moins une substance active de médicament, ainsi que des particules de polyvinylpyrrolidone réticulée, et dans lequel, en ce qui concerne l'auto-adhésif, il s'agit d'un auto-adhésif thermofusible, produit au moyen d'un procédé selon une des revendications 8 à 12.
